# EUROPÄISCHE PATENTSCHRIFT

(11) **EP 0 884 097 B1**
(45) Veröffentlichungstag und Bekanntmachung des Hinweises auf die Patenterteilung: **07.11.2001**
(21) Anmeldenummer: 98110345.0
(22) Anmeldetag: 05.06.1998
(51) Int. Cl.: B01J 2/26, C07C 67/52, B01D 9/00

(54) **Verfahren zur Herstellung von verbackungsfreien Hydroxipivallinsäureneopentylglykolester-Granulaten**
Method for making hydroxypivalic acid neopentylglycol ester non-caking granulates
Procédé de fabrication de granulats de l'ester néopentylglycol de l'acide hydroxypivalique ayant des propiétés d'anti-mottage

(30) Priorität: 10.06.1997 DE 19724461
(43) Veröffentlichungstag der Anmeldung: 16.12.1998
(73) Patentinhaber: BASF AKTIENGESELLSCHAFT, 67056 Ludwigshafen (DE)
(72) Erfinder: Melder, Johann-Peter, Dr., 67141 Neuhofen (DE); Baumann, Dieter, 67227 Frankenthal (DE); Maltry, Bernhard, 67283 Obrigheim (DE)
(74) Vertreter: Isenbruck, Günter, Dr.

(56) Entgegenhaltungen:
- EP-A- 0 210 415
- EP-A- 0 492 148
- EP-A- 0 829 298
- DE-B- 1 222 891

## Beschreibung

Die Erfindung betrifft ein Verfahren zur Herstellung von verbackungsfreien Hydroxipivalinsäureneopentylglykolester-Granulaten und die Verwendung einer Granuliervorrichtung zur Herstellung der Granulate.

Hydroxipivalinsäureneopentylglykolester (HPN) wird unter anderem als Bestandteil von Innenbeschichtungen von Konservendosen verwendet, um die Schlagzähigkeit der Innenbeschichtung zu erhöhen. Zur Konfektionierung von HPN wird üblicherweise eine HPN-Schmelze über einen Tropfenformer auf ein Kühlband aufgebracht. Dabei entstehen flache schuppenförmige Granulate, die bei der Lagerung in Gebinden zum Verbacken neigen.

Das Verbacken erschwert dabei die Dosierung und Entnahme der Granulate aus den Gebinden.

Aus der DE-A-35 22 359 (EP-A-0 210 415) ist ein Verfahren zum Konfektionieren von kristallinen organischen Materialien, wie Hydroxipivalinsäureneopentylglykolester, bekannt, wobei ein Produkt mit verbesserter Rieselfähigkeit erhalten wird. Dabei wird in einer zweiwelligen Schneckenmaschine mit im gleichen Sinne rotierenden Schneckenwellen ein pulverförmiges Material verdichtet oder ein schmelzflüssiges Material kristallisiert und verdichtet, bei Massetemperaturen, die etwa 1 bis 20°C unterhalb des Schmelzpunktes des eingesetzten Materials liegen.

Das Material wird sodann durch eine beheizte Lochplatte in eine Zone mit niedrigerem Druck ausgestoßen. Die Lochplatte wird auf eine Temperatur von 1 bis 30°C oberhalb des Schmelzpunktes des Materials erwärmt, so daß die an der Wandung der Lochplatte vorbeigeführten Einzelkristalle zu einem Film aufschmelzen, welcher nach seiner Erstarrung ein festes Korsett für das kompaktierte kristalline Material bildet. Die extrudierten Materialstränge werden in einer nachfolgenden Zone zerkleinert und gekühlt. Die Rieselfähigkeit der so erhaltenen Granulate ist nicht für alle Anwendungen ausreichend.

Die prioritätsältere, nicht vorveröffentlichte EP-A 0 829 298 betrifft ein Verfahren zur Herstellung von Hydroxypivalinsäureneopentylglykolester-Granulaten durch Aufbringen einer Hydroxypivalinsäureneopentylglykolester-Schmelze auf eine Kühlfläche, auf der die Schmelze erstarrt. Dabei enthält die Schmelze mindestens 3 Gew.-%, bezogen auf die Gesamtmenge an Hydroxypivalinsäureneopentylglykolester, an Hydroxypivalinsäureneo-pentylglykolester-Kristallen. Die Schmelze kann als flächige Schicht auf die Kühlfläche aufgebracht und nach dem Erstarren zu Granulaten zerkleinert werden, oder die Schmelze kann tropfenweise auf die Kühlfläche aufgebracht werden. Das Verfahren führt zu verbackungsarmen Granulaten.

Aufgabe der vorliegenden Erfindung ist die Bereitstellung eines Verfahrens zur Herstellung von HPN-Granulaten, das zu verbackungsarmen Granulaten führt, und die Nachteile bekannter Verfahren vermeidet.

Diese Aufgabe wird erfindungsgemäß gelöst durch ein Verfahren zur Herstellung von Hydroxypivalinsäureneopentylglykolester-Granulaten durch Aufbringen einer Hydroxipivalinsäureneopentylglykolester-Schmelze auf eine Kühlfläche, auf der die Schmelze erstarrt, das dadurch gekennzeichnet ist, daß die Hydroxypivalinsäureneopentylglykolester-Schmelze als flächige Schicht auf die Kühlfläche aufgebracht und nach dem Erstarren zu Granulaten zerkleinert wird und die Schmelze weniger als 3 Gew.-%, bezogen auf die Gesamtmenge an Hydroxipivalinsäureneopentylglykolester, an Hydroxypivalinsäureneopentylglykolester-Kristallen enthält.

Mit Hilfe des erfindungsgemäßen Verfahrens ist es möglich, HPN-Granulate herzustellen, die in Gebinden keine Neigung zu Verbackungen zeigen, da die Granulatteilchen eine große Härte und geringe Plastizität aufweisen.

Bei Granulaten, die aus mit Kristallen geimpften Schmelzen oder dünnen Schichten hergestellt werden, ist das Gefüge aus in der Schmelze vorliegenden und dort weiter wachsenden Kristalliten aufgebaut. Der Festkörper weist schließlich viele Korngrenzen auf, die gegeneinander verschiebbar sind und ihm Plastizität verleihen. Bei Druckbelastung werden die Kontaktstellen zwischen den Partikeln abgeplattet und so vergrößert, wodurch sich große Haftkräfte durch Kohäsion aufbauen. Bei der Erstarrung einer HPN-Schicht aus einer kristallarmen oder vorzugsweise kristallfreien Schmelze entsteht ein Festkörper mit einer Gefügestruktur von hohem Ordnungsgrad. Dadurch weisen aus dieser erstarrten Schicht gebrochene Granulate eine große Härte und geringe Plastizität auf. Hierdurch wird eine Abplattung der partikulären Kontaktstellen in Gebinden (Haufwerken) vermieden. Der Aufbau von Haftkräften durch Kohäsion kann so vermieden werden.

Vorzugsweise enthält die Schmelze vor dem Abkühlen weniger als 1 Gew.-%, besonders bevorzugt weniger als 0,5 Gew.-%, bezogen auf die Gesamtmenge an HPN, an HPN-Kristallen. Insbesondere ist die Schmelze im wesentlichen oder vollständig frei von HPN-Kristallen. Die Schmelze wird vor dem Aufbringen auf die Kühlfläche wird vorzugsweise in einem Vorkühler auf eine Temperatur nahe des Schmelzpunktes abgekühlt. Für HPN liegt diese Temperatur in der Nähe von 50°C. Der Durchlauf durch den Vorkühler kann so eingestellt werden, daß keine Kristalle in der Schmelze am Ausgang des Vorkristallisators vorliegen.

Vorzugsweise wird die Schmelze so auf die Kühlfläche, vorzugsweise ein Kühlband, aufgebracht, daß die Dicke der flächigen Schicht mindestens 2 mm beträgt. Besonders bevorzugt beträgt die Dicke der flächigen Schicht 5 bis 20 mm. Nach dem vollständigen Erstarren der flächigen Schicht wird diese dann in einem Brecher zerkleinert.

Die Erfindung wird nachstehend anhand der Zeichnung weiter erläutert, in der

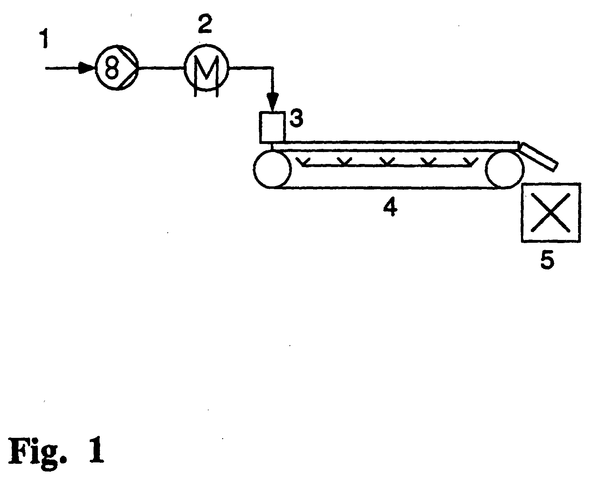

Figur 1 eine Vorrichtung für die Herstellung von HPN-Granulaten zeigt, in der
- 1: HPN-Schmelze
- 2: Vorkühler
- 3: Flächenaufgabesystem
- 4: Kühlband
- 5: Brecher
bedeutet.

Die erfindunggemäß verwendete Vorrichtung ist an sich bekannt. Eine derartige Vorrichtung ist beispielsweise in der DE-A-28 47 887 beschrieben. Nähere Angaben zu verwendbaren Flächenaufgabesystemen sind dort beschrieben.

Gemäß der in Figur 1 abgebildeten Ausführungform wird die HPN-Schmelze (1) zunächst durch einen Vorkühler (2) geführt. Die heiße flüssige HPN-Schmelze wird im Vorkühler (2) auf eine Temperatur in der Nähe des Schmelzpunktes (50°C) abgekühlt. Dann wird die vorgekühlte Schmelze über ein Flächenaufgabesystem (3) auf ein kontinuierlich angetriebenes Kühlband (4) aufgebracht und erstarrt auf diesem.

Das Flächenaufgabesystem (3) ist so gestaltet, daß die Schmelze über im wesentlichen die gesamte Breite des Kühlbandes (4) verteilt wird. Hierzu kann das Kühlband mit einer Randbegrenzung versehen sein, um über die gesamte Breite des Bandes eine gleichmäßige Schichtdicke zu erzielen. Die Schichtdicke der so gebildeten flächigen Schicht beträgt vorzugsweise minde stens 2 mm, besonders bevorzugt 5 bis 20 mm, insbesondere 5 bis 15 mm.

Am Bandende des im Kreis geführten Bandes bricht die erstarrte Schicht beispielsweise aufgrund der Schwerkraft ab und wird in einem Brecher (5) weiter zerkleinert. Hierbei werden flächige Granulate mit einem mittleren Teilchendurchmesser von 3 bis 50 mm, vorzugsweise 4 bis 30 mm, insbesondere 5 bis 20 mm erhalten. Diese HPN-Granulate sind vorzugsweise quaderähnlich beziehungsweise isometrisch geformt Die erhaltenen HPN-Granulate zeigen eine sehr geringe Neigung zum Verbacken.

Die Erfindung betrifft weiterhin die Verwendung einer Granuliervorrichtung für eine Schmelze, umfassend nacheinander über Zuleitungen verbunden einen Vorkühler (2), ein Flächenaufgabesystem (3) zum Aufbringen der Schmelze auf ein nachfolgendes Kühlband (4), an das sich ein Brecher (5) zur Granulierung von Hydroxipivalinsäureneopentylglykolester anschließt. Die Schmelze ist dabei wie vorstehend angegeben aufgebaut.

Die erfindungsgemäßen Granulate weisen eine höhere Schüttdichte und bessere Dosierbarkeit auf als bekannte Granulate. Insbesondere die im wesentlichen sphärischen Granulate sind leicht dosierbar und haben eine sehr hohe Schüttdichte.

## Patentansprüche

1. Verfahren zur Herstellung von Hydroxipivalinsäureneopentyglykolester-Granulaten durch Aufbringen einer Hydroxipivalinsäureneopentylglykolester-Schmelze auf eine Kühlfläche, auf der die Schmelze erstarrt, **dadurch gekennzeichnet, daß** die Hydroxipivalinsäureneopentylglykolester-Schmelze als flächige Schicht auf die Kühlfläche aufgebracht und nach dem Erstarren zu Granulaten zerkleinert wird und die Schmelze weniger als 3 Gew.-%, bezogen auf die Gesamtmenge an Hydroxipivalinsäureneopentylglykolester, an Hydroxipivalinsäureneopentylglykolester-Kristallen enthält.

2. Verfahren nach Anspruch 1, **dadurch gekennzeichnet, daß** die Schmelze weniger als 1 Gew.-%, bezogen auf die Gesamtmenge an Hydroxipivalinsäureneopentylglykolester, an Hydroxipivalinsäureneopentyglykolester-Kristallen enthält

3. Verfahren nach Anspruch 1 oder 2, **dadurch gekennzeichnet, daß** die Schmelze im wesentlichen frei von Hydroxipivalinsäureneopentylglykolester-Kristallen ist.

4. Verfahren nach einem der Ansprüche 1 bis 3, **dadurch gekennzeichnet, daß** die Dicke der flächigen Schicht mindestens 2 mm beträgt.

5. Verfahren nach Anspruch 4, **dadurch gekennzeichnet, daß** die Dicke der flächigen Schicht 5 bis 20 mm beträgt.

6. Verfahren nach einem der Ansprüche 1 bis 5, **dadurch gekennzeichnet, daß** die Hydroxipivalinsäureneopentylglykolester-Schmelze zunächst durch einen Vorkühler und dann über ein Flächenaufgabesystem auf ein kontinuierlich angetriebenes Kühlband aufgebracht wird, auf diesem erstarrt und nach Verlassen des Kühlbandes in einem Brecher in Granulatform gebracht wird.

7. Verwendung einer Granuliervorrichtung für eine Schmelze, umfassend nacheinander über Zuleitungen verbunden einen Vorkühler (2), ein Flächenaufgabesystem (3) zum Aufbringen der Schmelze auf ein nachfolgendes Kühlband (4), an das sich ein Brecher (5) anschließt, zur Granulierung von Hydroxipivalinsäureneopentylglykolester, **dadurch gekennzeichnet, daß** die Granuliervorrichtung mit einer Hydroxipivalinsäureneopentylglykolester-Schmelze beschickt wird, die weniger als 3 Gew.-%, bezogen auf die Gesamtmenge an Hydroxipivalinsäureneopentylglykolester, an Hydroxipivalinsäureneopentylglykolester-Kristallen enthält.

## Claims

1. A process for the production of neopentyl glycol hydroxypivalate granules in which a neopentyl glycol hydroxypivalate melt is deposited on a chill surface, on which the melt solidifies, which comprises depositing the neopentyl glycol hydroxypivalate melt, which contains less than 3% by weight, based on the total amount of neopentyl glycol hydroxypivalate, of neopentyl glycol hydroxypivalate crystals, on the chill surface as a flat film, which, after solidification, is comminuted to give granules.

2. A process as claimed in claim 1, wherein the melt contains less than 1% by weight, based on the total amount of neopentyl glycol hydroxypivalate, of neopentyl glycol hydroxypivalate crystals.

3. A process as claimed in claim 1 or 2, wherein the melt is essentially free from neopentyl glycol hydroxypivalate crystals.

4. A process as claimed in any one of claims 1 to 3, wherein the thickness of the flat film is at least 2 mm.

5. A process as claimed in claim 4, wherein the thickness of the flat film is from 5 to 20 mm.

6. A process as claimed in any one of claims 1 to 5, wherein the neopentyl glycol hydroxypivalate melt is firstly passed through a precooler and is then deposited, via a deposition system, on a continuously driven chill belt, solidifies thereon, and, after leaving the chill belt, is converted into granules in a breaker.

7. The use of a granulation device for a melt, comprising, connected in series via feed lines, a precooler (2), a deposition system (3) for depositing the melt on a subsequent chill belt (4), which is followed by a breaker (5) for granulation of neopentyl glycol hydroxypivalate, wherein the granulation device is charged with a neopentyl glycol hydroxypivalate melt which contains less than 3% by weight, based on the total amount of neopentyl glycol hydroxypivalate, of neopentyl glycol hydroxypivalate crystals.

## Revendications

1. Procédé de préparation de granulats de néopentylglycolester de l'acide hydroxypivalique par application d'une masse fondue de néopentylglycolester de l'acide hydroxypivalique sur une surface froide, sur laquelle la masse fondue durcit, **caractérisé en ce que** la masse fondue du néopentylglycolester de l'acide hydroxypivalique est appliquée en couche plane sur la surface froide et, après durcissement, est broyée en granulés, et que la masse fondue contient moins de 3% en poids, par rapport à la quantité totale de néopentylglycolester de l'acide hydroxypivalique, de cristaux de néopentylglycolester de l'acide hydroxypivalique.

2. Procédé selon la revendication 1, **caractérisé en ce que** la masse fondue contient moins de 1% en poids, par rapport à la quantité totale de néopentylglycolester de l'acide hydroxypivalique, de cristaux de néopentylglycolester de l'acide hydroxypivalique.

3. Procédé selon la revendication 1 ou 2, **caractérisé en ce que** la masse fondue est essentiellement exempte de cristaux de néopentylglycolester de l'acide hydroxypivalique.

4. Procédé selon l'une des revendications 1 à 3, **caractérisé en ce que** l'épaisseur de la couche plane est d'au moins 2 mm.

5. Procédé selon la revendication 4, **caractérisé en ce que** l'épaisseur de la couche plane est de 5 à 20 mm.

6. Procédé selon l'une des revendications 1 à 5, **caractérisé en ce que** la masse fondue de néopentylglycolester de l'acide hydroxypivalique passe d'abord dans un pré-refroidisseur, puis est appliquée par un système d'application en nappe sur une bande de refroidissement entraînée en continu, durcit sur celle-ci et, lorsqu'elle quitte la bande de refroidissement, est réduite en granulés par un broyeur.

7. Utilisation d'un dispositif de granulation pour une masse fondue, comprenant, successivement reliés par des lignes d'amenées, un pré-refroidisseur (2), un système d'application en nappe (3) pour l'application de la masse fondue sur une bande de refroidissement (4) en aval, à laquelle succède un broyeur (5) pour la granulation du néopentylglycolester de l'acide hydroxypivalique, **caractérisé en ce que** le dispositif de granulation est alimenté d'une masse fondue de néopentylglycolester de l'acide hydroxypivalique contenant moins de 3% en poids, par rapport à la quantité totale de néopentylglycolester de l'acide hydroxypivalique, de cristaux de néopentylglycolester de l'acide hydroxypivalique.
